# EUROPEAN PATENT APPLICATION

(11) **EP 1 466 597 A1**
(43) Date of publication of application: **13.10.2004**
(21) Application number: 03290863.4
(22) Date of filing: 07.04.2003
(51) Int. Cl.: A61K 31/23, A61K 35/60, A61P 9/10

(54) **Use of dha esters to control or prevent cardiovascular diseases**

(71) Applicant: Clinigenetics, 30000 Nîmes (FR)
(72) Inventor: Marguerie, Gérard, 94400 Vitry sur Seine (FR); Malaud, Eric, 30900 Nîmes (FR); Bishay, Karine, 30000 NImes (FR); Soulez, Marielle, 30000 Nîmes (FR)
(74) Representative: Galup, Cédric

(57) **Abstract**

The present invention relates to the use of esterified docosahexanoic acid, DHA, for the preparation of a medicament to inhibit the accumulation of lipid vesicles in macrophage and block the formation of foam cells.

## Description

The present invention relates to the use of esterified docosahexanoic acid, (DHA), in its lyso-phosphatidylcholine form, (lyso-PCDHA), or phosphatidylcholine form, (PCDHA), for the preparation of a medicament to inhibit the accumulation of lipid vesicles in macrophage and to block the formation of foam cells.

The invention is based on the observation that purified lyso-PCDHA and/or PCDHA, can inhibit the accumulation of lipid vesicles in macrophage and block the formation of foam cells. Phosphatidylcholine-containing-DHA derivatives are much more efficient than the free DHA form in this function.

This particular and unique activity demonstrates that phosphatidylcholine-containing-DHA derivatives may be useful in preventing or reducing the accumulation of foam cells at vascular sites that are prone to the development of an atherosclerotic plaque and may be used as pharmaceutical agents for treating atherosclerosis and related cardiovascular diseases.

Atherosclerosis is the most important cause of cardiovascular diseases and deaths in the industrialized countries (Ross R., 1993, Nature, 362, 801-809). Coronary atherosclerosis is responsible for over 500 000 deaths annually in the United States and for a vast number of other clinical complications.

Atherosclerosis is the result of a complex unbalanced cellular and molecular reaction, which normally functions as a defense mechanism in response to vascular injury. In pathological situations, however, this mechanism leads to endothelium dysfunction, cellular changes in the arterial intima and the continuous formation and growth of an arterial plaque containing lipids and foam cells.

The composition of vascular lesions that precede an ischemic episode are morphologically well characterized. These lesions are often clinically silent and are progressively established in a temporal sequence of early and advanced lesions named Type I, II, and III (Stary H. C., et al., 1994, Circulation, 89, 2462-2478).

Type I, II and III lesions are successive stages. Each type is characterized by the accumulation of lipoproteins and cholesterol esters associated with macrophages and macrophage foam cells to constitute fibro-fatty streaks. The formation and growth of these fibro-fatty streaks is also associated with the disruption of the intimal architecture of the plaque.

At very advanced stage, the plaque is invaded by apoptotic lipid-loaded foam cells. Type I, II and III lesions are regarded as silent precursors of atherosclerosis. Macrophages and macrophages loaded with lipid droplets are the major cellular component of an atheroma. Their proliferation and apoptosis lead to plaque rupture that induces the acute and ultimate phase of atheromatous diseases involving the formation of platelet aggregates. Although clinically silent in most circumstances, these plaques can be quantified by non-invasive imagery in the aorta and the coronary arteries of hyperlipidemic patients.

Mechanisms controlling plaque growth and erosion and plaque rupture leading to thrombotic episodes are largely unknown, and there is an unmet need for drugs in this area. The process appears to be the result of conflicting mechanisms. This includes for instance, lipid deposition and removal, cellular survival and death, cellular adhesion and extracellular matrix degradation and motility.

Atherosclerosis is initiated at specific sites by endothelium injury and dysfunction. Production of oxidized lipoproteins (oxLDL) and other oxidative and cytotoxic agents is probably the initial event that causes vascular injury. These agents have been shown to stimulate both survival and pro-apoptotic mechanisms in endothelial cells and macrophages. These initial reactions occur during hyperlipidemia, dyslipidemia, hypertension, diabetes and fluctuating shear stress.

Endothelial dysfunction creates a chronic inflammation, which results in a continuous recruitment of monocytes and macrophages. While beneficial in normal circumstances, this phenomenon may become pathologic and contributes to arterial plaque destabilization. The process is a slow reaction when compared to monocyte recruitment during infection, and may last a life time period.

Activated endothelial cells and monocytes express scavenger receptors such as CD36 or LOX1 that bind and uptake modified LDL. This reaction leads to the formation of foam cells, destabilization of the arterial plaque and causes plaque rupture resulting in acute thrombosis.

The existence of endothelium dysfunction and the perpetuation of lipid deposition and foam cells accumulation are the most important consequences of vascular lesions in patients at risk. Particularly, the abundance of oxLDL is an important factor of atherosclerosis.

Many of the drugs that are directed against these phenomenons, intend to treat the causes of atherosclerosis and the late ischemic event of atheromatous diseases. But there is a need for drugs that can treat the consequences of lipid deposition by controlling plaque growth and stability.

The development of an arterial plaque is complex and requires the expression of many genes with multiple functions in different cells including endothelial cells, macrophages and smooth muscle cells. The recruitment and differentiation of macrophages and the intracellular accumulation of lipid rich vesicles are recognized to be the major factor that is responsible for the spreading, the changes of the architecture and the instability of an atherosclerotic plaque. Lesions in the aortic roots are macrophage foam cells rich rather than smooth muscle cell-rich. Therefore, a need of molecules that can control the formation of foam cells exists.

The present invention is based on the demonstration, for the first time, that purified lyso-phosphatidylcholine docohexanoic acid (lyso-PCDHA) can block the accumulation of lipid rich vesicles in macrophage and prevent the formation of foam cell.

The present invention therefore has an important aim that of providing specific fatty acid based drugs, which can be administered very effectively, orally and which have and increased efficacy in the treatment of atherosclerosis and its complications.

According to the present invention, esterified docohexanoic acid (esterified DHA) derivatives and most particularly the lyso-phosphatidylcholine DHA (lyso-PCDHA) or the phosphatidylcholine DHA (PCDHA), can be used, eventually in their purified form, as pharmaceutical agents to prevent or to reduce the growth of an atherosclerotic plaque.

Esterified docosahexanoic acid, (esterified-DHA, or e-DHA), in its lyso-phosphatidylcholine form, (lyso-PCDHA), or phosphatidylcholine form, (PCDHA), have been described in EP 0 623 019 as platelet aggregation inhibitors, because of their anti-thromboxane A2 effect.

But as explained previously this effect is clearly different from the object of the instant invention, as, in atheromatous disease, platelet thrombi appear at the ultimate acute phase during plaque rupture episode and independently from foam cells accumulation.

Therefore, the object of the invention is the use of esterified docosahexanoic acid (DHA) derivatives as active ingredients for the preparation of a medicament to block the formation of foam cells, particularly the formation of macrophage foam cells, by inhibiting the accumulation of lipid vesicles.

In one particular embodiment, the esterified docosahexanoic acid (DHA) derivatives may be used in the preparation of a medicament to prevent or to inhibit the growth, the erosion, the rupture and/or the stability of an atherosclerotic plaque.

In one particular embodiment of the invention, the esterified docosahexanoic acid is selected from the group consisting of
- Esterified-DHA in the form of lyso-phosphatidylcholine (lyso-PCDHA);
- Esterified-DHA in the form of phosphatidylcholine (PCDHA) in which DHA is esterified in position sn-2 and which have an acyl group of 2 to 6 carbon atoms in position sn-1; and
- triglycerids in which DHA is esterified in position sn-2 and which have acyl groups of 2 to 6 carbon atoms in positions sn-1 and sn-3.

Preferably, the esterified docosahexanoic acid used in the invention is the lyso-phosphatidylcholine, (lyso-PCDHA), esterified in position sn-2.

In an other embodiment of the invention, the acyl group is selected from the group consisting of acetyl, propionyl, and butyryl. Preferably, the acyl group is butyryl.

The active ingredient used in the invention, the esterified docosahexanoic acid (esterified-DHA), and its derivatives as previously described, or a mixture of them, may be present in the medicament in a proportion comprised from 10% to 100% in weight of the total composition. Preferably, it is present in a proportion comprised from 10% to 70 % in weight of the total composition.

The medicament, which is prepared according to the invention, may be packaged in all the known galenic form compatible with its use, for example in a form acceptable with an administration *per os.* Preferably, the medicament is in a form for oral administration.

### Brief description of the figures:

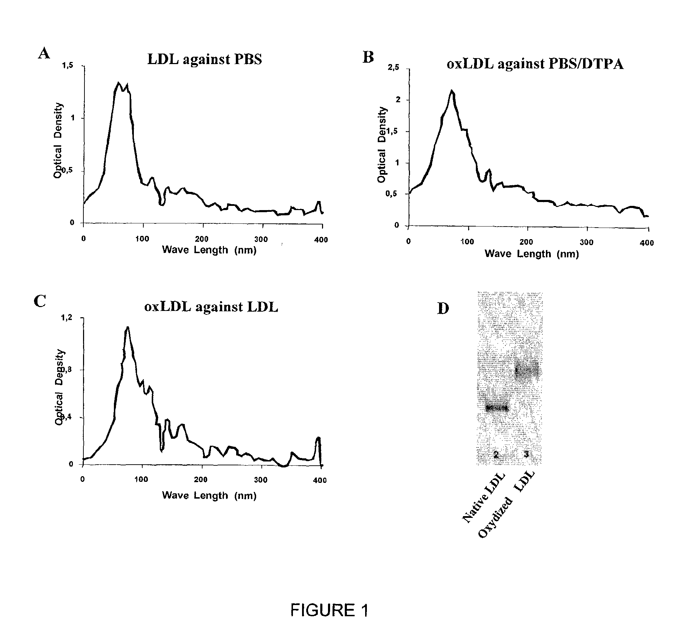

Figure 1: Characterization of the purified lipoproteins:
   A, B and C: absorption spectra between 200 nm and 400 nm of LDL (100 micrograms per ml) against PBS, oxLDL (100 micrograms per ml) against PBS/DTPA (diethylentriaminpentaacetic acid), and oxLDL (100 micrograms per ml) against LDL (100 micrograms per ml).
   D: electrophoretic mobility on agarose gel of native LDL and oxLDL.

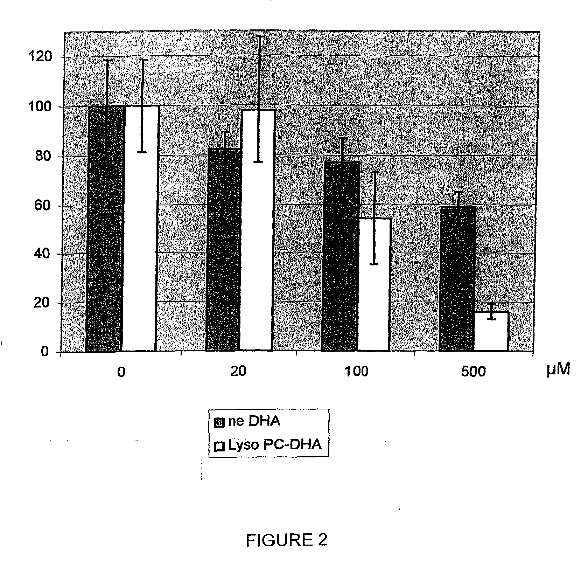

Figure 2: Dose dependent effect for the inhibition of lipid vesicles in macrophage cells by lyso-phosphatidylcholine-DHA (lyso-PCDHA) compared to the purified non esterified DHA molecule (ne-DHA). Foam cells were PMA stimulated THP1 cells in the presence of oxidized LDL. Inhibition of the formation of lipid vesicles was evaluated by the fluorescence density of labeled lyso-phosphatidylcholine DHA.

### Example 1 - Cell culture:

A series of primary or permanent cell lines can be used to monitor the effect of phosphatidylcholine DHA or lyso-phosphatidylcholine DHA on the atherogenic phenotype. Cells that can incorporate lipoprotein, modified lipoprotein including oxydized, acetylated lipoproteins, triglycerides, chilomicron and to exhibit vesicles that are characteristic of an atherosclerotic plaque associated foam cell. This includes but not limited to, U937, KG1, THP1, HUVEC, and smooth muscle cells.

In the present example, THP1 cells were used to generate a cell system that can mimic the formation of a foam cell in the plaque.

The THP-1 cell line from the European Collection of Cell Cultures, (ECACC, Wilshire, UK) were selected to generate a cellular model that mimics the differentiation and the growth of a foam cell. Typically, the cells (5.10⁵ cells/ml) were maintained and grown in RPMI-1640, 10% FBS, 100 Unit/ml penicillin and 100 µg/ml streptomycin, 200 mM L-Glutamine (Biowhittaker, Verviers , Belgium) in 37°C, 5% CO2 incubator. Medium was replaced every 2-3 days.

### Example 2 - Isolation and modification of lipoproteins:

Human LDL were isolated from fresh plasma using a two steps KBr gradient ultracentrifugation (Leger et al., Free Rad. Res., 2002, 36, 127-142), LDL were dialyzed against NaCl 150 mM, sodium phosphate 10 mM, DTPA 10 µM (pH 7,4) for 24 hours. Copper oxidized LDL was prepared under sterile conditions by incubating 0.2 mg/ml of LDL with 5µM CuSO4 for 16 hours at 37°C. At the end of this incubation, oxidation was stopped by addition of BHT (40 µM final) and DTPA (100 µM final). OxLDL were extensively dialyzed against NaCl 150 mM and Sodium Phosphate10 mM (pH 7,4) for 24 hours. All preparations were filtered through 0.4 µm filters.

LDL and oxLDL were extensively characterized by measuring the concentration of ApoB, total proteins, total cholesterol and vitamin E, the apparition of conjugated dienes (DO at 234 nm) and the determination of fatty acid (see table 1).

Finally, lipoproteins were also characterized by their electrophoretic mobility. Labeling of OxLDL with Cyanine 3 succinimidyl ester (Amersham Pharmacia Biotech) was prepared as described (Stanton et al., JBC, 11992, 267, 22446-22451). At the end of the labeling procedure, Cy3-OxLDL was extensively dialyzed and labeling efficiency was evaluated by measuring the absorbance at 548 nm.

### Example 3 - Inhibition of lipoprotein uptake:

To induce differentiation into macrophages, 1.5 10⁵ cells/wells were plated in 96-well plates in culture medium supplemented with 10⁻⁷ M of phorbol 12-myristate-13-acetate (Sigma) for 24 hours at 37°C, 5% CO₂. After one hour culture in RPMI 1640, 1%FBS, cells were incubated 2 hours with cy3 labeled oxLDL (1 µg/ml) in the presence or in the absence of DHA in its lyso-PCDHA form or in its non esterified form. Cells were washed twice with PBS and nucleus were stained with 20 µg/ml Hoechst 33342 for 10 minutes at room temperature. After 2 washes with PBS, images of Cy-3 oxLDL uptake were captured using a fluorescence microscope coupled with a CCD camera. Each image was analyzed and quantified using QFluoro Software (Leica).

### Example 4 - Preparation and purification of lyso-phosphatidylcholine DHA (LPCDHA)

At first, phosphatidylcholine-containing-DHA in position sn-2 can be purified from either fish oil extract or from the alcoholic extract of the biomes of a heterotrophic dinoflagellate *Cryptocodinium conhii,* according to the procedure described in patents EP 0 298 787 and US 5,654,290. The phosphatidylcholine containing DHA is isolated from the polar lipids by High Performance Liquid Chromatography (HPLC) according to the procedure described by Christie (Christie W.W., 1985, J. Lipid. Res., 26, 507-512).

The acyl chain in position sn-1 of the phosphatidylcholine-containing-DHA are hydrolyzed by any lipase having a phospholipase A: sub.1 activity. The Lyso-PCDHA obtained can be purified by chromatography on silica gel 60G according to Polette (Polette A. et al., 1999, Lipids, 34, 1333-1337). The purity of 1-lyso, 2-DHA-glycero-phosphocholine (LPCDHA) can be checked by HPLC and was routinely greater than 99%, with greater than 94% lyso-PCDHA esterified at position sn-2.

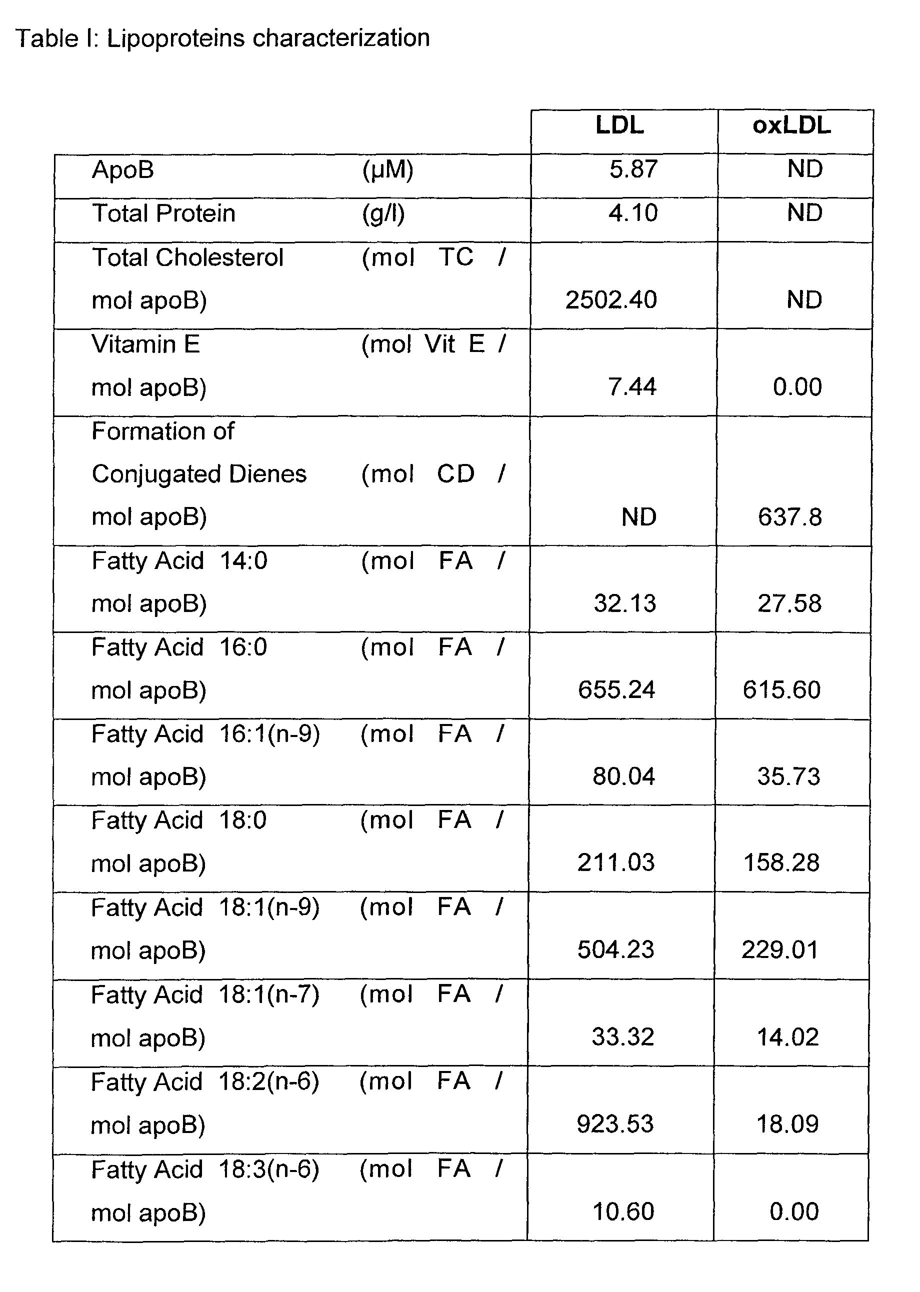

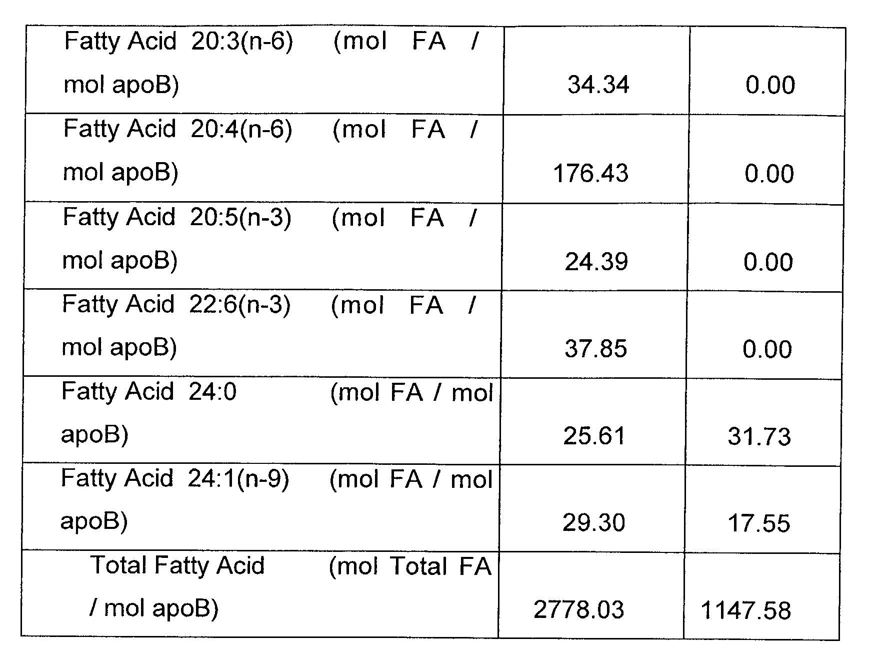

## Claims

1. Use of esterified docosahexanoic acid (DHA) as active ingredient for the preparation of a medicament to block the formation of foam cells.

2. Use according to claim 1, in the preparation of a medicament to block the formation of macrophage foam cells.

3. Use according to claim 1 or 2, in the preparation of a medicament to inhibit the accumulation of lipid vesicles in macrophage.

4. Use according to any one of the claims 1 to 3, in the preparation of a medicament to prevent or to inhibit the growth, the erosion, the rupture and/or the stability of an atherosclerotic plaque.

5. Use according to any one of claims 1 to 4, **characterized in that** the esterified docosahexanoic acid is selected from the group consisting of
- esterified-DHA in the form of lyso-phosphatidylcholine (lyso-PCDHA);
- esterified-DHA in the form of phosphatidylcholine (PCDHA) in which DHA is esterified in position sn-2 and which have an acyl group of 2 to 6 carbon atoms in position sn-1; and
- triglycerids in which DHA is esterified in position sn-2 and which have acyl groups of 2 to 6 carbon atoms in positions sn-1 and sn-3.

6. Use according to claim 5, **characterized in that** the esterified docosahexanoic acid used in the invention is the lyso-phosphatidylcholine (lyso-PCDHA) esterified in position sn-2.

7. Use according to claim 5, **characterized in that** the acyl group is acetyl, propionyl, or butyryl.

8. Use according to claim 7, **characterized in that** the acyl group is butyryl.

9. Use according to any one of claims 1 to 8, **characterized in that** the esterified docosahexanoic acid, and its derivatives, or a mixture of them, is present in the medicament in a proportion comprised from 10% to 100% in weight of the total composition.

10. Use according to claim 9, **characterized in that** the esterified docosahexanoic acid, and its derivatives, or a mixture of them, is present in the medicament preferably in a proportion comprised from 10% to 70 % in weight of the total composition.

11. Use according to any one of the claims 1 to 10, **characterized in that** the medicament is packaged for oral administration.
